# EUROPEAN PATENT APPLICATION

(11) **EP 4 245 755 A1**
(43) Date of publication of application: **20.09.2023**
(21) Application number: 22163423.1
(22) Date of filing: 22.03.2022
(51) Int. Cl.: C07D 251/60

(54) **PROCESS FOR THE PRODUCTION OF HIGH-PURITY MELAMINE FROM UREA PYROLYSIS, WITH REDUCED ENERGY CONSUMPTION AND IMPROVED INTEGRATION WITH THE UREA PLANT**

(30) Priority: 14.03.2022 IT 202200004898
(71) Applicant: EUROTECNICA MELAMINE AG, 8832 Wollerau (CH)
(72) Inventor: DE AMICIS, Alberto, I-20097 San Donato Milanese (MI) (IT); DI RUOCCO, Giuseppe, I-23900 Lecco (IT); FLORINDI, Americo, I-20025 Legnano (MI) (IT); PETRILLO, Francesca, I-20161 Milano (IT); BOGOTTO, Mattia, I-20148 Milano (IT)
(74) Representative: De Gregori, Antonella

(57) **Abstract**

An improved process is described for the production of high-purity melamine from the pyrolysis of urea, with reduced energy consumption and improved integration with the urea plant.

## Description

The present invention relates to an improved process, with reduced energy consumption, for the production of high-purity melamine by the high-pressure pyrolysis of urea, and improved integration with the urea plant.

More specifically, the invention relates to a process which provides for the purification in an aqueous ammonia solution of the melamine produced in the pyrolysis reactor, and its separation by crystallization.

It is known that the transformation of molten urea into melamine is described by the following overall reaction (1):

6 NH₂CONH₂ → (CN)₃(NH₂)₃ + 6 NH₃ + 3 CO₂ (1)

urea melamine ammonia carbon dioxide according to which for every kilogram of melamine, 1.86 kilograms of NH3 and CO₂ are formed, globally referred to as off-gas.

One of the most common industrial processes, based on the high-pressure pyrolysis of urea is that described in US patent 3,161,638 in the name of Allied. The process described by the above-mentioned patent is penalized by a high consumption of raw materials and services (so-called "utilities" such as steam, cooling water, fuel gas, electricity) and is jeopardized by an inefficient integration with the urea plant upstream due to the characteristics of the streams recycled to the same.

The solution of some of the drawbacks of the above-mentioned process is described in the state of the art represented by US patents 7,125,992 and EP 2385043 (corresponding to ITMI201000810) in the name of the same Applicant, a solution which provides for a decrease in energy consumption, investment and specific consumption of urea necessary for the production of melamine.

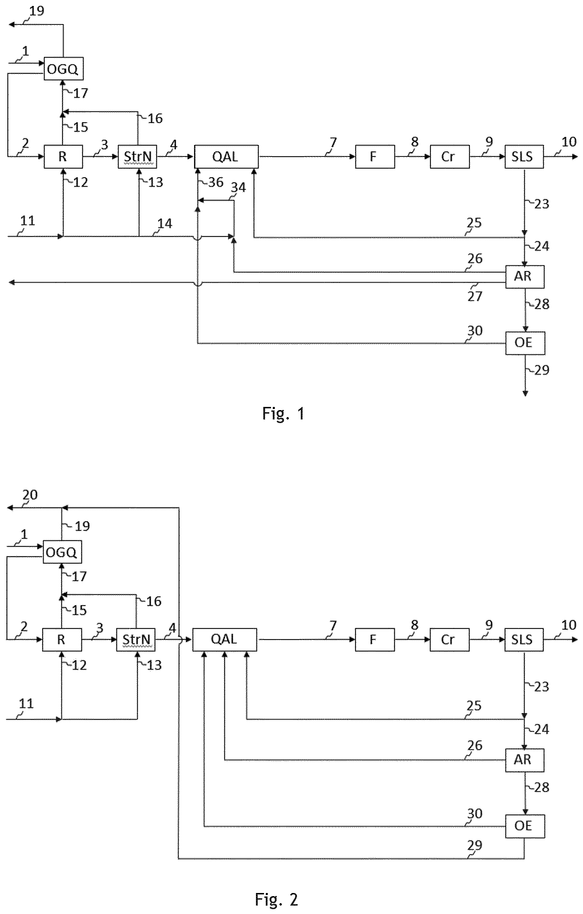

The process according to the state of the art is illustrated in the block diagram in figure 1.

According to the scheme, the urea (stream 1) produced in a synthesis plant (not shown in figure 1) adjacent to the melamine plant, is fed in the liquid state, at a temperature of 135 - 145°C, to a scrubbing section of the off-gases (OGQ) to recover the melamine vapour contained in the anhydrous off-gases (stream 19).

The use of molten urea allows practically all of the melamine contained in the anhydrous off-gases to be recovered.

The section OGQ operates at approximately the same pressure as the reaction section R, whereas the scrubbing temperature is lower than the reaction temperature. The scrubbing temperature is a compromise between the necessity of avoiding the degradation of the urea to undesired products and the necessity of avoiding the conversion of urea to melamine inside the scrubbing section OGQ.

In the scrubbing with molten urea, the melamine contained in the off-gases is removed and brought into solution/suspension in the molten urea. The stream of molten urea (stream 2) is fed to the reaction section R, the anhydrous off-gas stream (stream 19) is recycled to the urea plant.

The reaction section R is composed of a pyrolysis reactor which operates in continuous and in which a suitable heating system supplies the reacting system with the necessary reaction calories, maintaining the temperature within the range of 360 to 420°C. The reaction pressure is kept at a value above 70 bars.

Anhydrous gaseous NH3 (stream 12), is preferably also introduced, together with the molten urea, into the reactor.

Inside the reaction section, or again in the stripping section with NH₃ (StrN) downstream, the biphasic liquid/gas effluent produced by the pyrolysis reaction of urea to melamine is separated into a liquid stream 3 of raw melamine comprising unreacted urea, NH3, CO₂ and impurities such as OATs and polycondensates, and a first anhydrous off-gas stream 15, comprising NH3 and CO₂, saturated with melamine vapour.

The liquid stream 3 of raw melamine is sent to a stripping section StrN, which preferably operates under the same temperature and pressure conditions as the reaction section R, where it is put in contact with a stream 13 of anhydrous gaseous NH3. The stream of anhydrous gaseous NH3 flows in close contact with the liquid stream of raw melamine and extracts the CO₂ dissolved therein.

Furthermore, the permanence of the raw melamine with NH3 under the stripping conditions of CO₂, accelerates the conversion of unreacted urea to melamine, accelerates the conversion of OATs to melamine and favours the conversion of polycondensates to melamine.

A liquid melamine stream (stream 4) therefore exits from the section StrN which is not only practically free of CO_{2,} but is also partially purified as it is practically free of urea and contains reduced quantities of OATs and polycondensates.

A second anhydrous off-gas stream (stream 16) also exits from the section StrN, mainly comprising NH₃, a reduced amount of CO₂ and a certain quantity of melamine vapour.

The anhydrous off-gas streams from the sections R and StrN are subjected, together or separately, to a melamine recovery treatment by scrubbing with urea, in a single scrubbing section OGQ or in separate scrubbing sections (not represented in figure 1). The second anhydrous off-gas stream 16 is preferably combined with the first anhydrous off-gas stream 15, forming a single anhydrous off-gas stream 17 to be subjected to the same treatment in a single scrubbing section with urea OGQ.

The liquid stream 4 of melamine is sent from the stripping section StrN to a quench-ammonolysis section QAL, where the quenching treatment (dissolution of the raw melamine in ammonia water) and ammonolysis treatment (elimination of the polycondensates) are effected. The section QAL can be composed of one or more stationing devices, preferably a single device. The stream 4 in this apparatus is put in close contact with an aqueous ammonia solution (stream 36), in which it is totally dissolved at a temperature of 160 - 180°C, preferably of 170 - 172°C.

The aqueous stream 36 is formed by the combination of an aqueous stream (stream 30) coming from the subsequent steps of the purification and recovery process of the melamine and a stream of NH3 (stream 34), formed in turn by the combination of the stream 26 of NH3, also coming from the subsequent steps of the process and a make-up stream of NH3 14. An aqueous direct recycling stream of the crystallization mother liquor is also sent to the quench-ammonolysis section QAL (stream 25).

In the quench-ammonolysis section QAL, the residence time is sufficient for almost completely eliminating the polycondensates present in the stream 4, mostly transforming them into melamine. In the section QAL, the mass ratios between the different incoming streams are such as to have an incoming stream 7 having concentrations of NH₃ ranging from 10% to 17% by mass (preferably from 12% to 15% by mass) and melamine concentrations ranging from 5% to 19% by mass (preferably from 7% to 15% by mass, and more preferably from 9% to 11% by mass).

The stream 7 is sent to the filtration section F, composed of finishing filters which also complete the ammonolysis treatment. The outgoing stream from the filtration section F (stream 8) is sent to the crystallization section Cr, where the melamine is precipitated by lowering the temperature to about 40 - 50°C, obtaining an aqueous suspension of high-purity melamine (stream 9).

The stream 9 leaving the section Cr is subjected to a solid/liquid separation in the section SLS, whereas the melamine crystals (stream 10) are separated from the crystallization mother liquor (stream 23) and sent to the drying and packaging sections (not indicated in figure 1).

The stream of crystallization mother liquor 23, exiting from the separator SLS, is therefore divided into two streams (streams 24 and 25): stream 25 is directly recycled to the quench-ammonolysis section QAL, whereas stream 24 is sent to the deammoniation section AR.

In the deammoniation section AR, the stream 24 is separated into three streams:
a stream 26 of pure NH3 to be recycled to the ammonolysis section QAL;
a stream 27 rich in CO₂;
an aqueous stream 28 comprising melamine, OATs and free of CO₂ and NH3.

The deammoniation section AR can operate according to any known method for the separation of water-NH₃-CO₂ mixtures.

The stream 27 rich in CO₂ can be sent directly to the urea plant, or recycled to a point in the melamine plant; it is preferably recycled to a point where the CO₂ contained therein does not lower the pH in the crystallization step and can, ultimately, leave the melamine plant, taking with it less water than in the case of being sent directly to the urea plant as mentioned above (for example, after a treatment that reduces its water content).

The deammoniated mother liquor stream 28, i.e. comprising melamine, OATs and free of CO₂ and NH₃, is sent to a section OE for the elimination of the OATs and to obtain an aqueous solution to be recycled to the quench-ammonolysis section QAL (stream 30).

The section OE can operate in many different modes. The two preferred modes are already applied in the state of the art and are the following:
in one case, the OATs are crystallized by cooling and neutralization with CO₂ and then separated from the stream 28 by ultrafiltration;
in another case, the whole stream 28 is sent for decomposition obtaining a stream of water, NH₃ and CO₂ (stream 29) substantially free of impurities, which can be discharged or reused in a suitable point of the plant, and a stream of water 30 to be recycled to the section QAL.

The process described above is currently applied industrially in numerous plants, but is nevertheless jeopardized by various drawbacks:
- a high consumption of steam and cooling water due to the necessity of treating the non-recyclable part of the mother liquor, stream 24 in the deammoniation section AR in order to obtain a pure ammonia stream, stream 26;
- the need for recycling the stream 27 to the urea plant which, in addition to being rich in CO_{2,} contains water. As is known, water has a negative effect on the conversion of the reagents NH3 and CO₂ to urea and it is therefore preferable to minimize the water content in the streams recycled from the melamine plant to the urea plant;
- the need for importing a quantity of ammonia from the battery limits in order to replenish the ammonia in the section QAL recycled to the urea plant via stream 27.

The objective of the present invention is therefore to define a process for the production of melamine which overcomes the drawbacks previously indicated.

A further objective of the present invention is to define a process for the production of melamine, improved with respect to the state of the art, which allows a substantially economical recovery and recycling of the ammonia and process water contained in the mother liquor and which allows one or more streams containing CO₂ and NH₃, free of or with a low water content, to be recycled to the whole urea synthesis plant.

The present invention therefore relates to a process for the production of high-purity melamine with high yields by the pyrolysis of urea carried out at a temperature ranging from 360 to 450°C and at a pressure higher than 7 MPa, wherein the product of the urea pyrolysis reaction, consisting of a gas phase (15) and a liquid phase (3), is subjected to subsequent treatments, wherein:
- the liquid phase (3) leaving the pyrolysis Reactor (R) containing melamine, unreacted urea, oxidized intermediate pyrolysis products (OATs) and deammoniation and condensation products of the melamine (polycondensates), is sent to a subsequent reaction step in a reactor or Post-Reactor (StrN) which operates under temperature and pressure conditions substantially equal to those of the pyrolysis Reactor (R) and wherein a stream of gaseous, anhydrous and superheated NH3 is fed under pressure (13), in order to eliminate the dissolved CO₂, complete the urea pyrolysis reaction and transform most of the OATs into melamine, obtaining a liquid phase of melamine (4) and a second gas phase (16);
- the anhydrous gaseous phase coming from the pyrolysis Reactor (15) and the anhydrous gaseous phase coming from the Post-Reactor (16) are combined and the off-gas gaseous stream thus obtained (17) is subjected to a scrubbing (OGQ) with molten urea (1) for the recovery of the melamine contained therein as vapour, before said scrubbed off-gas gaseous stream (19) is sent back to a urea synthesis process for the recovery of the NH₃ and CO₂ contained therein;
- the liquid phase leaving the Post-Reactor (4) is brought into aqueous solution in a quench-ammonolysis section (QAL), in the presence of NH3, keeping the whole solution under controlled conditions of temperature and residence time, preferably at a temperature ranging from 160 to 180°C, more preferably from 170 to 172°C, at a pressure ranging from 2.5 to 4.5 MPa, more preferably equal to 2.5 MPa, for a time ranging from 30 to 60 minutes, more preferably 45 minutes, obtaining a solution substantially free of polycondensates (7) which is subjected to crystallization (Cr) to give high-purity melamine (10) and mother liquor (23) containing melamine and reduced quantities of OATs, a part of said mother liquor (25) being recycled, without any treatment, to the quenching step in the quench-ammonolysis section (QAL);
- the remaining part of the mother liquor (24) not recycled to the quench-ammonolysis section (QAL) is treated in a deammoniation section (AR) from which a stream containing water and ammonia (26) is obtained by distillation sent to the quench-ammonolysis section (QAL) and a stream (28) containing water, CO₂ and OATs, practically free of NH3, which is sent to the subsequent elimination phase of the OATs in the elimination section of the OATs (OE);
- said elimination step of the OATs produces a liquid phase (30) substantially composed of water which is recycled to the quench-ammonolysis section (QAL) and a gaseous phase (29), comprising NH₃ and CO₂ saturated with water, coming from the elimination section of the OATs (OE) wherein said gas phase (29)
- is combined with the scrubbed off-gas stream (19) before being sent back (20) to a urea synthesis process for the recovery of the NH3 and CO₂ contained therein, or
- is recycled to the deammoniation section (AR), obtaining a liquid phase (31) containing NH3, CO₂ and water, which is combined with the scrubbed off-gas stream (19) before being sent back (20) to a urea synthesis process for the recovery of the NH₃ and CO₂ contained therein, or
- is recycled to the deammoniation section (AR), obtaining a liquid phase (31) containing NH3, CO₂ and water, which is sent back to a urea synthesis process for the recovery of the NH₃ and CO₂ contained therein.

Furthermore, the gaseous phase (29) coming from the elimination section of the OATs (OE) is joined with the scrubbed off-gas gaseous stream (19), and the stream thus obtained can alternatively be sent to a condensation step in an off-gas condensation section (OGC) before being sent back (20) to a urea synthesis process for the recovery of the NH₃ and CO₂ contained therein.

The liquid phase (31) containing NH3, CO₂ and water, is combined with the scrubbed off-gas stream (19) and the stream thus obtained can alternatively be sent to a condensation step in an off-gas condensation section (OGC) before being sent back (20) to a urea synthesis process for the recovery of the NH3 and CO₂ contained therein.

The scrubbing step (OGQ) of the off-gas stream (17) combined with molten urea (1) is effected at the same pyrolysis pressure of the urea in the Reactor (R).

The elimination step of the OATs is preferably a decomposition reaction of the OATs carried out at the same pyrolysis pressure of the urea in the Reactor (R) and scrubbing of the off-gas stream (17) combined with molten urea (1) in the off-gas scrubbing section OGQ.

The process according to the present invention has simple but substantial modifications of the process according to the state of the art, which result in substantial advantages.

In the attached figures 1-4, figure 1 is representative of a process according to the state of the art (US 7,125,992), whereas figures 2-4 are representative of different embodiments of the process according to the present invention.

The block diagram of figure 2 substantially shows the main sections and flow lines of the process according to the present invention.

In the block diagram of the process according to the present invention (figure 2), the same references have been used for the sections and the same numbers for the streams also used in the block diagram of the state of the art (figure 1) in order to make a comparison between the process scheme according to the state of the art and the process scheme according to the present invention easier and more immediate.

### Deammoniation section (AR):

In the process according to the present invention, the part of mother liquor (stream 24) not recycled to the quench-ammonolysis section (QAL) is treated in the deammoniation section (AR), in order to obtain a stream 26, composed of NH₃ and recycled water, which is sent to the quench-ammonolysis section (QAL) and a stream 28, containing water, CO₂ and OATs, practically free of NH₃, sent to the subsequent OATs elimination section (OE).

The substantial difference between the process according to the present invention and the process according to the state of the art consists in the fact that in the process of the state of the art the deammoniation section (AR) generates a stream of pure liquid ammonia to be recycled to the quench-ammonolysis section ( QAL) (stream 26 of figure 1). In order to obtain a stream of pure liquid ammonia, the deammoniation section (AR) is compelled to operate at a pressure that allows the condensation of pure ammonia at the temperature of the cooling water available in the process. This operating pressure of the distillation column of ammonia from the mother liquor also constrains the operating temperature of the column and consequently also the temperature of the steam fed to the reboiler of the column itself.

The process scheme according to the present invention does not provide that the deammoniation section (AR) produce a pure liquid ammonia stream: in the deammoniation section (AR) according to the scheme of the present invention, a distillation step is instead effected, of a stream composed of NH₃ and water and a stream containing water, CO₂ and OATs, practically free of NH₃.

The process according to the present invention, which does not require the formation of a stream of pure liquid ammonia from the deammoniation section AR, makes it possible to operate at a pressure lower than the pressure at which the distillation column operates according to the state of the art. Furthermore, the operating pressure of the distillation column according to the present invention is not constrained by the temperature of the cooling water available in the plant, consequently the operating pressure and temperature of the distillation column can be lower than those of the same column in the state of the art, with a consequent saving of investment on the column and steam consumption, as it is possible to use steam at a lower temperature.

A further important advantage of the deammoniation section AR of the process according to the present invention is that, contrary to the process of the state of the art, it does not provide for the production at the outlet of the stream 27 containing CO_{2,} NH3 and water, stream 27 which must then be recycled to the urea plant, where it must be treated to convert the NH3 and CO₂ reagents into urea. This stream 27 has the major drawback of containing a high water content and, as is well known, water does not favour the conversion reaction of NH₃ and CO₂ to urea. From the point of view of the urea plant, and therefore the integration of the urea plant with the melamine plant, the process according to the present invention which does not provide for the formation in the deammoniation section (AR) of a stream 27 of liquid carbamate, containing CO_{2,} NH3 and water, represents an important improvement in the effectiveness of the conversion of the reagents NH₃ and CO₂ into urea.

### Elimination section of the OATs OE:

In the process according to the present invention, as shown in figure 2, a stream 28, comprising water, CO₂ and OATs, practically free of NH3, exits from the deammoniation section AR and enters the elimination section of the OATs OE.

This stream 28, in the process according to the present invention, is treated in order to obtain a stream 30 of pure water and a stream 29 in vapour phase, comprising NH₃ and CO₂ saturated with water. The stream of pure water 30 is recycled to the quench-ammonolysis section QAL, whereas the stream 29, containing NH3 and water-saturated CO₂, joins the scrubbed off-gas stream 19 to form the off-gas stream 20 which is recycled to the Urea plant.

In the state of the art, the section OE can operate in two different modes a) and b):
In a first mode a), the OATs are crystallized by cooling and neutralized with CO₂, and then separated from the stream 28 by ultrafiltration, obtaining an aqueous stream 30 rich in melamine to be recycled to the section QAL and a stream rich in OATs to be sent for decomposition to obtain a stream 29 substantially free of impurities which can be discharged or reused in the plant.

Alternatively according to mode b), in the section OE the stream 28 is sent for decomposition to recover NH3 and CO₂, obtaining a stream 29 substantially free of impurities which can be discharged or reused in the plant, and a stream of water 30 to be recycled to the section QAL.

In the process according to the present invention, the elimination section of the OATs OE does not operate according to the methods described above a) and b) of the state of the art.

In the process according to the present invention, the section OE effects a decomposition reaction of the OATs carried out at the same pressure as the reaction section R and the scrubbing section of the off-gases OGQ. The water stream 30 to be recycled to the section QAL is obtained from this section OE, together with the stream 29 which is combined with the off-gas stream 19 coming from the scrubbing section OGQ, forming a stream 20 recycled to the urea plant.

The gaseous stream 29 is composed of NH₃ and CO₂ saturated with water and the lower the water content of the stream 29, the higher the operating pressure of the section OE will be.

The advantage of operating the section OE at the same pressure as the off-gas scrubbing section OGQ is that this allows the NH3 and CO₂ recovered from the decomposition of OATs to be recycled to the urea plant, reducing the consumption of raw materials such as NH₃ and CO₂. Furthermore, by operating at high pressure, the water content of stream 29 is extremely low with a consequent benefit in the conversion of NH3 and CO₂ into Urea in the urea plant.

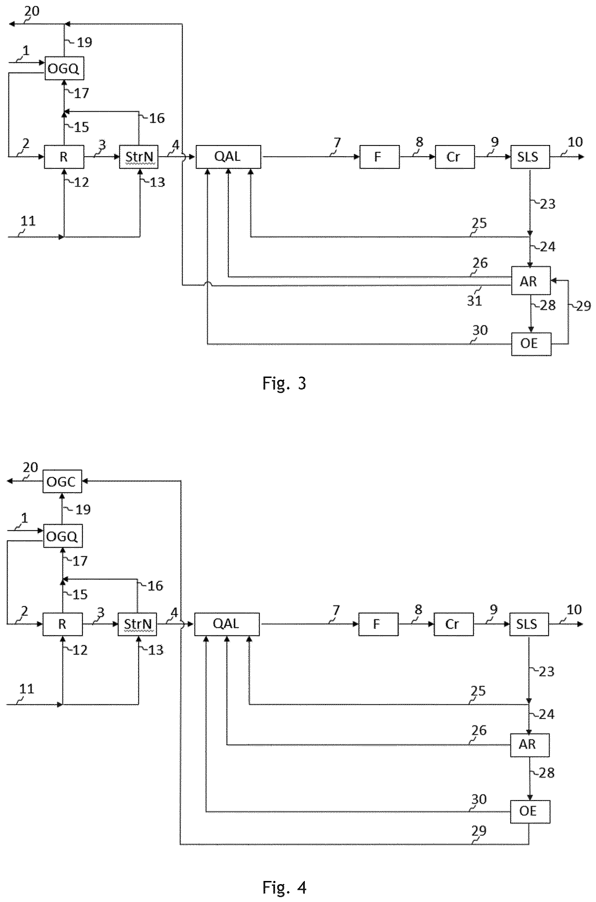

In the alternative embodiment of the process according to the present invention represented in figure 3, the gaseous stream 29 coming from the elimination section of the OATs (OE) and composed of NH₃ and CO₂ saturated with water, is instead recycled to the deammoniation section (AR) , obtaining a liquid phase 31, containing NH3, CO₂ and water, which is combined with the scrubbed off-gas stream (19) before being sent back (20) to a urea synthesis process for the recovery of the NH₃ and CO₂ contained therein. In a further alternative not shown in the figure, the gaseous stream 29 coming from the elimination section of the OATs (OE) and composed of NH₃ and CO₂ saturated with water, is recycled to the deammoniation section (AR), obtaining a liquid phase (31) containing NH3, CO₂ and water, which is sent back directly to a urea synthesis process for the recovery of the NH₃ and CO₂ contained therein.

In the alternative embodiment of the process according to the present invention shown in figure 4, the gaseous stream 29 coming from the elimination section of the OATs (OE) and composed of NH3 and CO₂ saturated with water, is combined with the scrubbed gaseous off-gas stream 19, and the stream thus obtained is sent to a condensation phase in an off-gas condensation section (OGC) before being sent back as stream 20 to a urea synthesis process for the recovery of NH₃ and CO₂ contained therein.

The process according to the present invention therefore has some surprising and undoubted advantages with respect to the processes of the state of the art and in particular, thanks to the modifications applied to the process steps carried out in the deammoniation section (AR), it is possible to operate the distillation column of the section AR at a pressure lower than the pressure at which the distillation column operates according to the state of the art, said pressure, moreover, no longer being constrained by the temperature of the cooling water available in the plant. The operating conditions, pressure and temperature, of the distillation column are consequently lower than those of the same column of the state of the art, with a consequent saving of investment on the column and a saving in terms of steam consumption, as it is possible to use steam at a lower temperature.

Thanks to the modifications applied to the process steps carried out in the deammoniation section (AR) and in the elimination section of the OATs (OE), which interact in a combined way, it has also been possible to obtain an important improvement in the integration of the urea plant with the melamine plant, in terms of the effectiveness of converting the NH3 and CO₂ reagents into urea: the liquid or gaseous streams fed to the urea synthesis process/plant for the recovery of the NH3 and CO₂ contained therein do not contain water or contain water in extremely reduced quantities (in any case much smaller than the processes of the state of the art) thus allowing an important improvement in the conversion of the NH3 and CO₂ reagents into urea.

Furthermore, again thanks to the modifications applied to the process steps carried out in the deammoniation section (AR) and in the elimination section of the OATs (OE), which interact in a combined way, it has been possible to obtain a reduction in the make-up ammonia (stream 11 ) compared to the state of the art.

The operating mode of the deammoniation section (AR) allows a stream 28 to be obtained, comprising water, CO₂ and OATs, practically free of NH₃. The make-up ammonia of this process is reduced by the same amount contained in the stream 27 of the state of the art, no longer present in the present process, said stream being recycled to the urea plant.

The ammonia contained in the stream 29 by the elimination section of the OATs (OE) is essentially that obtained from the decomposition of the OATs in the same section.

The reduction in the make-up ammonia has the positive effect of obtaining a molar ratio between NH3 and CO₂ recycled to the urea synthesis plant closer to the stoichiometric ratio of the conversion reaction of NH₃ and CO₂ to Urea. The advantage of having a ratio between NH₃ and CO₂ close to the stoichiometric ratio of the urea reaction allows a reduction in the recycling of ammonia and recoveries within the urea plant with a consequent reduction in energy consumption, consequently leading to an improvement in the interaction/integration of the melamine plant with the urea plant.

The present invention also relates to an apparatus for implementing the improved process for the production of melamine according to the present inventyion, comprising:
i) a separation section for separating a biphasic liquid/gas effluent produced in a urea pyrolysis reaction into a liquid stream of raw melamine (3) and a first stream of anhydrous off-gases (15) comprising NH3, CO₂ and melamine vapour, said separation section being connected to a urea pyrolysis reaction section (R) from which it receives said biphasic liquid/gas effluent, said separation section being inside or outside said reaction section (R);
ii) a stripping section (StrN) for putting said liquid stream of raw melamine (3) coming from said reaction section (R) in contact with a stream of anhydrous gaseous NH3 and forming a liquid stream of raw melamine depleted of CO₂ (4) and a second anhydrous off-gas stream (16) comprising NH3, CO₂ and melamine vapour, said stripping section (StrN) being connected to said reaction section (R) from which it receives said liquid stream of raw melamine (4);
iii) a scrubbing section (OGQ) for putting said first and second anhydrous off-gas streams (15,16) in contact with a stream of molten urea (1) and forming a stream (2) comprising melamine and urea and a scrubbed off-gas stream (19) comprising NH3 and CO₂, said scrubbing section (OGQ) being connected to said separation section from which it receives said first anhydrous off-gas stream (15), and to said stripping section (StrN ) from which it receives said second anhydrous off-gas stream (16);
iv) a quench-ammonolysis section (QAL) for eliminating the polycondensates from the raw melamine stream (4), in the presence of NH₃, and obtaining a solution (7) substantially free of polycondensates, said quench-ammonolysis section (QAL) being connected to said stripping section (StrN) from which it receives said raw melamine stream (4);
v) a melamine recovery section (Cr) for recovering the melamine contained in said solution (7) substantially free of polycondensates by means of crystallization by cooling, with the formation of a stream (10) of crystallized melamine and a stream (23) of mother liquor, said melamine recovery section (Cr) being connected to said quench-ammonolysis section (QAL) from which it receives said solution (7);
vi) a deammoniation section (AR) suitable for treating, by distillation, a part of the mother liquor coming from the melamine recovery section (Cr) with the formation of a stream containing water and ammonia (26) and a stream (28) containing water, CO₂ and OATs, practically free of NH₃, said deammoniation section (AR) being connected to said melamine recovery section (Cr) from which it receives the stream of mother liquor (24), and to said quench-ammonolysis section (QAL) to which it sends the stream containing water and ammonia (26);
vii) an elimination section of the OATs (OE) suitable for decomposing the OATs producing a liquid phase (30) substantially composed of water which is recycled to the quench-ammonolysis section (QAL) and a gas phase (29), comprising NH3 and CO₂ saturated with water, said elimination section of the OATs (OE) being connected to the deammoniation section (AR) from which it receives the stream (28) containing water, CO₂ and OATs, practically free of NH3, and to the quench-ammonolysis section (QAL) to which it sends the liquid phase (30) substantially composed of water.

In the apparatus according to the present invention, the scrubbing section (OGQ) can be connected to an off-gas condensation section (OGC) for the condensation of the off-gases, to which it feeds the scrubbed off-gas stream (19) comprising NH₃ and CO₂.

### EXAMPLE

950 kg of molten urea at a temperature of 135°C were introduced into the off-gas scrubbing section OGQ in a high-purity melamine production plant according to the present invention and comprising all the steps included in the process scheme represented in figure 2.

The off-gas scrubbing operation was carried out at a pressure of 8.0 MPa and at a temperature of 215°C.

Two streams were thus obtained from the off-gas scrubbing section OGQ: a stream 19 containing 713 kg/h of anhydrous off-gases, free of melamine and a stream 2, containing recovered urea and melamine, which is fed to the Reactor of the section R.

In the reactor, the temperature was maintained at 380°C and the pressure at 8.0 MPa for a residence time (referring to the incoming molten urea flow) of about 50 minutes.

Two streams were obtained from the Reactor: a stream 15 of CO₂ and NH₃ saturated with melamine which was sent to the off-gas scrubbing section OGQ for the recovery of the melamine, and a liquid stream 3 of raw melamine having a melamine titer of 91% weight.

This liquid stream was fed to the Post-Reactor of the section StrN where, under the same conditions as the Reactor, it was treated with a gaseous flow of 100 Kg/h of superheated anhydrous ammonia which almost completely eliminated the dissolved CO₂. The residence time of the liquid melamine in this apparatus was 45 minutes. The ammonia and gaseous CO₂ exiting from the Post-Reactor (stream 16) were combined with the off-gases 15 exiting from the Reactor and were fed together with the off-gas scrubbing section OGQ. A stream 4 of molten melamine was also obtained from the Post-Reactor containing about 6,000 ppm by weight of OATs and less than 1% by weight of polycondensates.

This stream 4 of purified melamine was fed to the Quenching Column of the section QAL, where the melamine was dissolved in an aqueous ammonia solution under pressure conditions of 2.5 MPa and at a temperature of 170°C. The NH3 concentration in the Quenching Column was kept at above 13% by weight.

The solution 7 thus obtained was fed to the filtration section F where the ammonolysis treatment was completed. The solution 8 at the outlet was found to contain 7.9% by weight of melamine, less than 2,500 ppm of OATs and a quantity of polycondensates of less than 10 ppm.

This stream 8 was brought to conditions of almost atmospheric pressure and a temperature of 45°C in the Crystallizer. Under these conditions, 320 kg/h of very pure melamine (titer 99.95% by weight) were crystallized, which were then separated in the Liquid/Solid Separator and dried.

3,702 kg/h of mother liquor were recovered in the Liquid/Solid Separator,, of which 3,200 kg/h (stream 25) were directly recycled to the Quenching column QAL (stream 25) in order to dissolve the melamine leaving the reaction system, whereas the remaining 502 kg/h (stream 24) were distilled in the Deammoniation Column AR where a stream 26 composed of 73 kg/h of ammonia and 4 kg/h of water was recovered and a stream 28 of 425 kg/h containing water, CO₂ and OATs, practically free of NH₃.

The stream 28 was fed to the elimination section of the OATs OE which operates at the pressure of the Reactor and Post-reactor 8.0 MPa. In the decomposer of the section OE, the OATs were decomposed to NH₃ and CO₂, and from this section a stream 29 of 4 kg/h of NH3 and CO₂ saturated with water in the vapour state and a stream 30 of 421 kg/h of pure water were obtained in the liquid state. The stream 29 was combined with the anhydrous off-gas stream 19 from the scrubbing section OGQ, generating the stream 20 which was recycled to the urea plant. The stream of pure water 30 was recycled to the Quenching Column of the section QAL in order to dissolve the melamine in solution.

## Claims

1. A process for the production of high-purity melamine with high yields by the pyrolysis of urea carried out at a temperature ranging from 360 to 450°C and at a pressure higher than 7MPa, wherein the product of the urea pyrolysis reaction, consisting of a gas phase (15) and a liquid phase (3), is subjected to subsequent treatments, wherein:
- the liquid phase (3) leaving the pyrolysis Reactor (R) containing melamine, unreacted urea, oxidized intermediate pyrolysis products (OATs) and deammoniation and condensation products of the melamine (polycondensates), is sent to a subsequent reaction step in a reactor or Post-Reactor (StrN) which operates under temperature and pressure conditions substantially equal to those of the pyrolysis Reactor (R) and wherein a stream of gaseous, anhydrous and superheated NH3 is fed under pressure (13), in order to eliminate the dissolved CO₂, complete the urea pyrolysis reaction and transform most of the OATs into melamine, obtaining a liquid phase of melamine (4) and a second gas phase (16);
- the anhydrous gaseous phase coming from the pyrolysis Reactor (15) and the anhydrous gaseous phase coming from the Post-Reactor (16) are combined and the off-gas gaseous stream thus obtained (17) is subjected to a scrubbing (OGQ) with molten urea (1) for the recovery of the melamine contained therein as vapour, before said scrubbed off-gas gaseous stream (19) is sent back to a urea synthesis process for the recovery of the NH₃ and CO₂ contained therein;
- the liquid phase leaving the Post-Reactor (4) is brought into aqueous solution in a quench-ammonolysis section (QAL), in the presence of NH3, keeping the whole solution under controlled conditions of temperature and residence time, preferably at a temperature ranging from 160 to 180°C, more preferably from 170 to 172°C, at a pressure ranging from 2.5 to 4.5 MPa, more preferably equal to 2.5 MPa, for a time ranging from 30 to 60 minutes, more preferably 45 minutes, obtaining a solution substantially free of polycondensates (7) which is subjected to crystallization (Cr) to give high-purity melamine (10) and mother liquor (23) containing melamine and reduced quantities of OATs, a part of said mother liquor (25) being recycled, without any treatment, to the quenching step in the quench-ammonolysis section (QAL);
- the remaining part of the mother liquor (24) not recycled to the quench-ammonolysis section (QAL) is treated in a deammoniation section (AR) from which a stream containing water and ammonia (26) is obtained by distillation sent to the quench-ammonolysis section (QAL) and a stream (28) containing water, CO₂ and OATs, practically free of NH₃, which is sent to the subsequent elimination phase of the OATs in the elimination section of the OATs (OE);
- said elimination step of the OATs produces a liquid phase (30) substantially composed of water which is recycled to the quench-ammonolysis section (QAL) and a gaseous phase (29), comprising NH₃ and CO₂ saturated with water, coming from the elimination section of the OATs (OE) wherein said gas phase (29)
- is combined with the scrubbed off-gas stream (19) before being sent back (20) to a urea synthesis process for the recovery of the NH3 and CO₂ contained therein, or
- is recycled to the deammoniation section (AR), obtaining a liquid phase (31) containing NH3, CO₂ and water, which is combined with the scrubbed off-gas stream (19) before being sent back (20) to a urea synthesis process for the recovery of the NH₃ and CO₂ contained therein, or
- is recycled to the deammoniation section (AR), obtaining a liquid phase (31) containing NH3, CO₂ and water, which is sent back to a urea synthesis process for the recovery of the NH₃ and CO₂ contained therein.

2. The process according to claim 1, wherein the gaseous phase (29) coming from the elimination section of the OATs (OE) is combined with the scrubbed off-gas gaseous stream (19), and the stream thus obtained is sent to a condensation step in an off-gas condensation section (OGC) before being sent back (20) to a urea synthesis process for the recovery of the NH₃ and CO₂ contained therein.

3. The process according to claim 1, **characterized in that** the liquid phase (31) containing NH3, CO₂ and water, is combined with the scrubbed off-gas stream (19) and the stream thus obtained is sent to a condensation step in an off-gas condensation section (OGC) before being sent back (20) to a urea synthesis process for the recovery of the NH₃ and CO₂ contained therein.

4. The process according to any of the previous claims, **characterized in that** the scrubbing step (OGQ) of the off-gas stream (17) combined with molten urea (1) is effected at the same pyrolysis pressure of the urea in the Reactor (R).

5. The process according to any of the previous claims, **characterized in that** the elimination step of the OATs is a decomposition reaction of the OATs carried out at the same pyrolysis pressure of the urea in the Reactor (R) and scrubbing of the off-gas stream (17) combined with molten urea (1) in the off-gas scrubbing section OGQ.

6. An apparatus for implementing the improved process for the production of melamine according to claim 1, comprising:
i) a separation section for separating a biphasic liquid/gas effluent produced in a urea pyrolysis reaction into a liquid stream of raw melamine (3) and a first stream of anhydrous off-gases (15) comprising NH₃, CO₂ and melamine vapour, said separation section being connected to a urea pyrolysis reaction section (R) from which it receives said biphasic liquid/gas effluent, said separation section being inside or outside said reaction section (R);
ii) a stripping section (StrN) for putting said liquid stream of raw melamine (3) coming from said reaction section (R) in contact with a stream of anhydrous gaseous NH3 and forming a liquid stream of raw melamine depleted of CO₂ (4) and a second anhydrous off-gas stream (16) comprising NH3, CO₂ and melamine vapour, said stripping section (StrN) being connected to said reaction section (R) from which it receives said liquid stream of raw melamine (4);
iii) a scrubbing section (OGQ) for putting said first and second anhydrous off-gas streams (15,16) in contact with a stream of molten urea (1) and forming a stream (2) comprising melamine and urea and a scrubbed off-gas stream (19) comprising NH₃ and CO₂, said scrubbing section (OGQ) being connected to said separation section from which it receives said first anhydrous off-gas stream (15), and to said stripping section (StrN ) from which it receives said second anhydrous off-gas stream (16);
iv) a quench-ammonolysis section (QAL) for eliminating the polycondensates from the raw melamine stream (4), in the presence of NH₃, and obtaining a solution (7) substantially free of polycondensates, said quench-ammonolysis section (QAL) being connected to said stripping section (StrN) from which it receives said raw melamine stream (4);
v) a melamine recovery section (Cr) for recovering the melamine contained in said solution (7) substantially free of polycondensates by means of crystallization by cooling, with the formation of a stream (10) of crystallized melamine and a stream (23) of mother liquor, said melamine recovery section (Cr) being connected to said quench-ammonolysis section (QAL) from which it receives said solution (7);
vi) a deammoniation section (AR) suitable for treating, by distillation, a part of the mother liquor coming from the melamine recovery section (Cr) with the formation of a stream containing water and ammonia (26) and a stream (28) containing water, CO₂ and OATs, practically free of NH₃, said deammoniation section (AR) being connected to said melamine recovery section (Cr) from which it receives the stream of mother liquor (24), and to said quench-ammonolysis section (QAL) to which it sends the stream containing water and ammonia (26);
vii) an elimination section of the OATs (OE) suitable for decomposing the OATs producing a liquid phase (30) substantially composed of water which is recycled to the quench-ammonolysis section (QAL) and a gas phase (29), comprising NH3 and CO₂ saturated with water, said elimination section of the OATs (OE) being connected to the deammoniation section (AR) from which it receives the stream (28) containing water, CO₂ and OATs, practically free of NH₃, and to the quench-ammonolysis section (QAL) to which it sends the liquid phase (30) substantially composed of water.

7. The apparatus according to claim 6, wherein the scrubbing section (OGQ) is connected to an off-gas condensation section (OGC) for the condensation of the off-gases, to which it feeds the scrubbed off-gas stream (19) comprising NH₃ and CO₂.
